(19) Europäisches Patentamt
European Patent Office
Office européen des brevets

(11) Numéro de publication: **0 264 306 B1**

(12) # FASCICULE DE BREVET EUROPEEN

(45) Date de publication de fascicule du brevet: **12.06.91**

(51) Int. Cl.5: **A61K 31/445**

(21) Numéro de dépôt: **87401980.5**

(22) Date de dépôt: **04.09.87**

(54) **(Naphtyl-2 méthoxy)-4 pipéridine pour le traitement de l'obésité.**

(30) Priorité: **10.09.86 FR 8612642**

(43) Date de publication de la demande: **20.04.88 Bulletin 88/16**

(45) Mention de la délivrance du brevet: **12.06.91 Bulletin 91/24**

(84) Etats contractants désignés: **BE CH DE FR GB IT LI LU NL SE**

(56) Documents cités:

**FR-A- 2 514 353**

**BR. J. CLIN. PHARMACOL., vol. 11, 1981, pages 96-98; R.J. SIMPSON et al.: "Effect of zimelidine, a new antidepressant, on appetite and body weight"**

**INT. J. OBESITY, vol. 5, 1981, pages 377-378; J. SMEDEGAARD et al.: "Treatment of obesity by femoxetine a selective 5 HT reuptake inhibitor"**

**LIFE SCI., vol. 43, no. 8, 1988, pages 651-658, Pergamon Press Plc, US; I. ANGEL et al.: "Anorectic activities of serotonin uptake inhibitors: correlation with their potencies at inhibiting serotonin uptake in vivo and 3H-mazindol binding in vitro"**

**DRUG DEVELOPMENT RESEARCH, vol. 12, no. 1, 1988, pages 29-30, Alan R. Liss, Inc.: B. SCATTON et al.: "A novel selective and potent serotonin uptake inhibitor"**

(73) Titulaire: **SYNTHELABO**
**58 rue de la Glacière**
**F-75013 Paris(FR)**

(72) Inventeur: **Langer, Salomon**
**92, rue Jouffroy**
**F-75017 Paris(FR)**

(74) Mandataire: **Thouret-Lemaitre, Elisabeth et al**
**SYNTHELABO Service Brevets 22, avenue Galilée**
**F-92352 LE PLESSIS ROBINSON CEDEX(FR)**

EP 0 264 306 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet l'utilisation de la (naphtyl-2 méthoxy)-4 pipéridine pour la fabrication de médicaments destinés à combattre l'obésité.

La (naphtyl-2 méthoxy)-4 pipéridine est décrite dans le certificat d'addition n° 81 19025 (2 514 353) du 9 octobre 1981 rattaché au brevet n° 80 09513 français.

Ce composé de formule

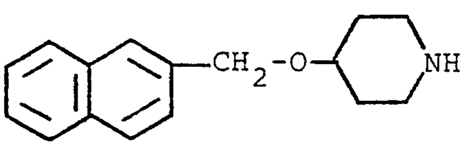

est préparé selon le mode opératoire décrit dans le C.A. n° 81 19025, c'est à dire par réaction entre le chlorure de naphtyl-2 méthyle et l'hydroxy-4 nitro-4 benzoyl-1 pipéridine, puis déprotection de l'azote de la pipéridine. Le fumarate de la (naphtyl-2 méthoxy)-4 pipéridine fond à 170-171,5 °C.

Ce composé avait été décrit dans le C.A. n° 81 19025 comme possédant des propriétés antidépressives.

La Demanderesse, en poursuivant ses recherches, a montré que la (naphtyl-2 méthoxy)-4 pipéridine possède des propriétés anorexigènes et peut être utilisée pour le traitement de l'obésité.

Les essais pharmacologiques suivants ont été effectués : on utilise des rats mâles Sprague-Dawley (Charles River, France) pesant 210 à 250 g ; on maintient les animaux dans une atmosphère lumineuse - sombre basée sur un cycle de 12h. On leur fournit de l'eau tout au long de l'expérience.

Avant les essais, les rats sont maintenus sans nourriture pendant 18 à 20h. Tous les essais commencent vers 9.00 - 10.00h le matin.
On injecte ou administre par gavage le produit à tester (1 ml/kg) dissout dans une solution salée.
On place les animaux dans des cages de plastique dans lesquelles on introduit la nourriture dans des boîtes de Petri prépesées 5, 10 ou 30 mn après l'administration du produit à étudier.

A des intervalles constants (0,5h) on retire les coupes de nourriture et la quantité de nourriture restante est pesée. La signification des données est analysée par les tests de Duncan ou de Dunnett.

A des doses de 1 à 10 mg/kg par voie intrapéritonéale le composé produit une inhibition de la prise de nourriture, fonction de la dose, pendant la première heure qui suit l'introduction de la nourriture.

L'effet maximal anorexigène est obtenu dans les 30 à 60 premières minutes qui suivent l'introduction de la nourriture.
La dose active 50 (dose qui inhibe de 50% la prise de nourriture pendant les 30 premières minutes) du composé est de 3,8 mg/kg par voie intrapéritonéale.
Lorsque le composé est administré par voie orale une inhibition marquée de la prise de nourriture est observée à des doses de 5 à 30 mg/kg.

La (naphtyl-2 méthoxy)-4 pipéridine apparaît être un agent anorexigène relativement puissant.
Elle peut dont être utilisée pour le traitement de l'obésité.

Les compositions pharmaceutiques contenant le composé en association avec tout excipient approprié pour l'administration par voie orale ou parentérale font partie de l'invention.
La posologie quotidienne peut aller de 10 à 50 mg.

## Revendications

1. Utilisation de la (naphtyl-2 méthoxy)-4 pipéridine pour la fabrication de médicaments destinés à combattre l'obésité.

## Claims

1. Use of 4-(2-naphtylmethoxy)piperidine in the manufacture of a medicament intended for controlling obesity.

## Ansprüche

1. Verwendung der 4-(2-naphtylmethoxy)-piperidine zur Herstellung eines Arzneimittels zur Bekämpfung der Fettleibigkeit.